# EUROPÄISCHE PATENTSCHRIFT

(11) **EP 2 808 458 B1**
(45) Veröffentlichungstag und Bekanntmachung des Hinweises auf die Patenterteilung: **25.10.2017**
(21) Anmeldenummer: 14001861.5
(22) Anmeldetag: 28.05.2014
(51) Int. Cl.: E03F 5/04

(54) **Wasserablaufvorrichtung für eine Dusche und Duschbodenelement**
Water drainage device for a shower and shower floor element
Dispositif d'écoulement d'eau pour une douche et élément de sol de douche

(30) Priorität: 29.05.2013 DE 102013105542
(43) Veröffentlichungstag der Anmeldung: 03.12.2014
(73) Patentinhaber: wedi GmbH, 48282 Emsdetten (DE)
(72) Erfinder: Wedi, Stephan, 48282 Emsdetten (DE)
(74) Vertreter: Träger, Denis

(56) Entgegenhaltungen:
- EP-A1- 2 586 921
- EP-A2- 2 532 795
- WO-A1-2007/004863
- DE-A1-102008 046 671

## Beschreibung

Die Erfindung betrifft eine Wasserablaufvorrichtung gemäß dem Oberbegriff des Anspruchs 1.

Weiterhin betrifft die Erfindung ein bodenebenes Duschbodenelement mit einer solchen Wasserablaufvorrichtung, gemäß dem Oberbegriff des Anspruchs 15. Aus EP 2 532 795 A2 ist eine Wasserablaufvorrichtung für einen besonders flachen Aufbau bekannt, aufweisend einen rinnenförmigen Ablaufbereich mit eingebautem, mittig angeordneten Geruchsverschluss, dessen sattelförmige Abdeckung zwei in einem Gehäuse des Geruchsverschlusses angeordnete Trennwände überbrückt. Jede Trennwand ist durch zwei nach oben spitz zulaufende Wandelemente gebildet. Die Trennwände des Geruchsverschlusses unterteilen sein Gehäuse in eine mittlere und zwei äußere Kammern.

Bei der Druckschrift WO 2007/004863 A1 ist in einem rinnenförmigen Drainagekanal ein Geruchsverschluss entnehmbar eingesetzt. An einer Seitenwand oder im Boden des Geruchsverschlusses ist ein Ablaufstutzen angeordnet, der in eine Ablaufrinne einführbar ist. Auch bei diesem Geruchsverschluss überbrückt eine Abdeckung zwei einander gegenüberliegende Seitenwände. Dadurch kann Wasser über die Seitenwände in den Innenraum des Geruchsverschlusses, und von dort aus durch den Ablaufstutzen in die Ablaufrinne fließen.

Eine weitere Wasserablaufvorrichtung und ein bodenebenes Duschbodenelement ist in DE 10 2008 046 671 A1 beschrieben. Das Duschbodenelement ist Teil eines Bodens des Feuchtraums, insbesondere der Dusche und wird mit einem rinnenförmigen Ablaufkörper zu einer Einheit vormontiert, wobei in den rinnenförmigen Ablaufkörper von oben eine Geruchsverschlusseinheit eingebracht ist. Die Konstruktion der Wasserablaufvorrichtung und insbesondere der Geruchsverschlusseinheit erfordert eine beachtliche Aufbauhöhe sowie auf Grund der kreisförmigen Kontur des Stutzens 4 einen erheblichen Abstand zwischen dem Ablaufsystem und einer Raumwand, vor die das Duschbodenelement eingebaut werden soll.

Die Druckschrift EP 2 586 921 A1 offenbart eine Duschwanne mit einem rinnenförmigen Ablaufkanal, der mit einer Rinnenabdeckung verschließbar ist. An der Rinnenabdeckung ist ein Vorsprung angeordnet, der zwischen einem inneren und einem äußeren Abschnitt eines Wasserablaufs einschiebbar ist und gemeinsam mit diesen einen Geruchsverschluss bildet. Der innere Abschnitt ist nach oben hin geöffnet und bildet den Wasserauslauf. Dadurch ist die Aufbauhöhe auch bei dieser Duschwanne erheblich. Dieses Dokument entspricht dem Oberbegriff des Anspruchs 1, und wird als nächstliegend Stand der Technik abgesehen. Aufgabe der Erfindung ist es, eine neuartige Wasserablaufvorrichtung für einen besonders flachen und wandnahen Aufbau zu konzipieren. Eine zusätzliche Aufgabe der Erfindung ist es, ein bodenebenes und wandnahes Duschbodenelement für eine solche Wasserablaufvorrichtung zu entwickeln.

Diese Aufgabe ist durch eine Wasserablaufvorrichtung der im Oberbegriff des 1. Anspruchs genannten Art gelöst, bei der
- das Glockenelement (39) einen langgestreckten, geradlinig verlaufenden Scheitelbereich (48), zwei ebenso langgestreckte Seitenwände (49, 50) und zwei an den Schmalseiten des Hohlkörpers liegende, weitere Seitenwände (55, 56) aufweist, die eine zweite Sperrwand (35) bilden,
- die mittlere Kammer (33) in Draufsicht auf den Boden (31) gänzlich von der zweiten Sperrwand (35), ausgenommen im Durchgangsbereich (57) zum Ablaufstutzen (14) umschlossen ist, so dass die Kammern (34, 33, 32) durch ringförmig umeinander herum liegende Sperrwände (36, 35) und die Gehäusewand (61) gebildet sind, und
- wobei die Sperrwand (35) des Glockenelementes (39) eine Aussparung (42) aufweist, so dass das Glockenelement (39) über die innere Kammer (34) und den von der inneren Kammer (34) ausgehenden Ablaufstutzen (14) stülpbar ist.

Die Sperrwand des Glockenelementes weist eine Aussparung auf, so dass das Glockenelement über die innere Kammer und den von der inneren Kammer ausgehenden Ablaufstutzen gestülpt werden kann. Es ist vorgesehen, dass die Aussparung im montierten Zustand den Ablaufstutzen dichtend umschließt. Dies ist insbesondere zur Einhaltung relevanter Normen, wie der DIN EN 1253/274, und nachfolgend für die baurechtliche Zulassung der Wasserablaufvorrichtung wichtig. Dementsprechend kann die Aussparung von einer Nut umgeben sein, in welche ein Dichtelement eingesetzt ist. Vorzugsweise ist die Aussparung und somit die Nut U-förmig. Das Glockenelement kann gegen Aufschwimmen durch Klick- oder Rastmittel oder anderes Sicherungsmittel arretiert werden.

Die strömungstechnischen Überlegungen ergaben, dass es besonders vorteilhaft ist, wenn die äußere Kammer auf Höhe einer Unterkante der Sperrwand eine erste Fläche und die mittlere Kammer eine zweite Fläche aufweist, deren Flächenverhältnis innerhalb eines Bereiches von 0,5 bis 0,8, vorzugsweise zwischen 0,63 und 0,69 liegt. Es hat sich erwiesen, dass eine vorteilhafte Sperrwaserhöhe, die einem Abstand zwischen der Oberkante der Sperrwand und deren Unterkante entspricht, mindestens 38 mm betragen soll. Diese Maßnahmen tragen zum sehr flachen Aufbau der Wasserablaufvorrichtung bei und ermöglichen gleichzeitig eine ausreichende Ablaufleistung des Gesamtsystems.

Besonders vorteilhaft ist es weiterhin für den ultraflachen Aufbau, wenn das Ablaufrohr der Wasserablaufvorrichtung innerhalb eines Umrisses einer Grundplatte des Duschbodenelementes liegt. Zu diesem Zweck kann an der Unterseite der Grundplatte eine nutartige Aussparung zur Aufnahme des Ablaufrohres vorgesehen sein.

Besonders vorteilhaft ist es weiterhin, wenn die Wasserablaufvorrichtung, ausgenommen die Rinnenabdeckung und das Glockenelement, in eine längliche, durchgehende Öffnung der besagten Grundplatte, auf deren Unterseite eingelassen und dort festgelegt, beispielsweise mit der Grundplatte verklebt ist. Dabei weist die längliche Öffnung der Grundplatte Ausmaße auf, die diejenigen der Rinnenabdeckung etwas überschreiten, so dass um die Rinnenabdeckung herum ein Spalt verlaufen kann. Eine spaltlose Ausführung ist auch möglich, wenn an der Rinnenabdeckung entsprechende Perforationen, wie Öffnungen, Schlitze oder gitterförmige Strukturen für den Wasserablauf eingebracht sind. Die Rinnenabdeckung selbst kann aus einer gitter- oder wabenförmigen Struktur; beispielsweise aus Streckmetall, gebildet sein.

Vorteilhaft für den Wasserablauf ist es, wenn die sich direkt an den Ablaufstutzen anschließende innere Kammer einen Boden aufweist, welcher beidseitig ein dem Ablaufstutzen zugerichtetes Gefälle aufweist oder muldenförmig ist. Dabei kann der Boden der inneren Kammer gegenüber dem restlichen Boden des Gehäuses angehoben sein.

Der Durchgangsbereich der inneren Kammer zum Ablaufstutzen kann auch trichterförmig gestaltet sein.

An den Ablaufstutzen kann ein kreisrundes oder ovales Ablaufrohr oder auch ein Vierkant-Ablaufrohr angeschlossen sein. Vorzugsweise weist der Ablaufstutzen einen rechteckigen Querschnitt auf, mit dem sich die Verbindung mit dem Vierkant-Ablaufrohr einfacher realisieren lässt.

Die Wasserablaufvorrichtung kann ein Rinnenmodul mit der Wasserauslauföffnung und ein Ablaufmodul mit einer Wassereinlauföffnung umfassen, wobei
- das Ablaufmodul das Geruchsverschlusssystem beinhaltet, aufweisend das oben erwähnte Gehäuse mit den Kammern und das Glockenelement,
- die Wasserauslauföffnung des Rinnenmoduls rechteckig oder oval ist,
- die Wassereinlauföffnung des Geruchsverschlusssystems eine zur jeweiligen Wasserauslauföffnung kompatible Kontur aufweist,
- zwischen dem Rinnenmodul und dem Ablaufmodul eine Verbindung vorgesehen ist, die eine Kleb-, Klick-, Rast- oder Schraubverbindung sein kann.

Das Rinnemodul und das Ablaufmodul können Kontaktflächen und Strukturelemente aufweisen, wobei die Strukturelemente so angelegt sind, dass sie nach Herstellung der Verbindung ein Dichtungslabyrinth ausbilden.

Es ist nicht ausgeschlossen, dass das Rinnenmodul mit dem Ablaufmodul, ausgenommen dem Glockenelement, in einem Materialstück, beispielsweise aus thermoplastischem Kunststoff gefertigt ist.

Es wird jedoch eine modulare Bauweise angestrebt, die sowohl den Anschluss eines herkömmlichen Standardablaufs mit einem zylindrischen, runden Ablauftopf als auch den Anschluss eines superflachen Ablaufs ermöglicht.

Zu diesem Zweck kann das Ablaufmodul mit wenigstens einem Adapter zur lösbaren Verbindung mit dem Rinnenmodul und unterschiedlichen Geruchsverschlusssystemen ausgestattet sein. Dabei ist der Adapter sowohl mit dem Rinnenmodul als auch mit der Wassereinlauföffnung des jeweiligen Ablaufmoduls wasserdicht verbindbar.

Ausführungsbeispiele der Erfindung sind anhand der Zeichnung näher erläutert.

Die Figuren zeigen:
- Fig. 1: ein Rinnenmodul mit sichtbarer zentralen Wasserauslauföffnung, in einer perspektivischen Ansicht von oben;
- Fig. 2: das Rinnenmodul gemäß Fig. 1, angeschlossen an ein separates Ablaufmodul, in einer perspektivischen Ansicht von unten;
- Fig. 3: einen in einem Materialstück mit dem Rinnenmodul ausgeführten Geruchsverschluss, ebenso in einer perspektivischen Ansicht von unten;
- Figuren 4 und 5: jeweils ein Rinnenmodul mit eingesetzter Rinnenabdeckung, in einer superflachen Ausführung und mit einem Standardablauf, in einer perspektivischen Ansicht von oben auf die Rinnenabdeckung;
- Figuren 6 und 7: die Rinnenmodule gemäß Figuren 4 und 5, jeweils mit einer Schnittdarstellung im Bereich des Ablaufstutzens;
- Figuren 8 und 9: Rinnenmodul mit superflachem Ablauf und einem Adaptermodul für den Standardablauf, in einer perspektivischen Ansicht von unten;
- Fig. 10: Detail einer Verbindung des Rinnenmoduls mit dem Ablaufmodul;
- Fig. 11: Anordnung der Wasserablaufvorrichtung in einem bodenebenen Duschbereich, in einer perspektivischen Ansicht von oben;
- Fig. 12: das Rinnenmodul in superflacher Ausführung mit angeschlossenem vierkantigen Ablaufrohr, in einer perspektivischen Ansicht von oben;
- Figuren 13 bis 15: das superflache Rinnenmodul, eingebaut in eine Grundplatte, mit eingesetzter und entnommener Rinnenabdeckung sowie mit entnommenem Glockenelement, jeweils in einer perspektivischen Ansicht von oben;
- Fig. 16: das in die Grundplatte eingebaute Rinnenmodul gemäß Fig. 12, in einem schematischen, axialen Schnitt durch das Ablaufrohr;
- Figuren 17 und 18: die Höhenverstellbarkeit der Wasserablaufvorrichtung, in einem schematischen axialen Schnitt durch das vierkantige Ablaufrohr;
- Figuren 19 und 20: Anordnung der Kammern und der Sperrwände in einer schematischen Draufsicht auf einen Boden des Gehäuses;
- Fig. 21: die modulare Wasserablaufvorrichtung in superflacher Ausführung, eingebaut in eine verflieste Grundplatte, in einem axialen Schnitt durch das Ablaufrohr;
- Fig. 22: einen Schnitt A - A gemäß Fig. 20 und
- Fig. 23: das Glockenelement in seiner einfachsten Form, perspektivisch dargestellt.

Begriffe, wie "oben", "obere", "unten", "untere" etc. beziehen sich auf die in eine Grundplatte eines Duschbodenelementes eingebaute Wasserablaufvorrichtung, wie dies auch in der Zeichnung dargestellt ist.

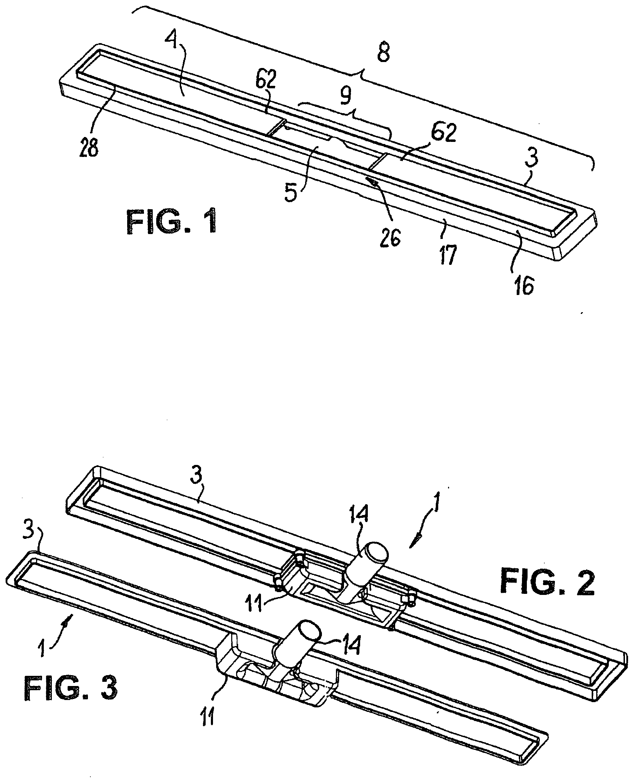

In Fig. 1 ist ein Rinnenmodul 3 gezeigt, aufweisend einen rinnenförmigen Ablaufbereich 8 und einen innerhalb des Ablaufbereichs 8 mittig liegenden, Übergangsbereich 9 mit einer rechteckigen Wasserauslauföffnung 5. Der durch einen umlaufenden, nach oben ragenden Kragen 28 begrenzte Ablaufbereich 8 ist spiegelsymmetrisch gegenüber der Wasserauslauföffnung 5 angeordnet und stellt eine rechteckige Wassereinlauföffnung 4 mit einem Gefälle 62 dar.

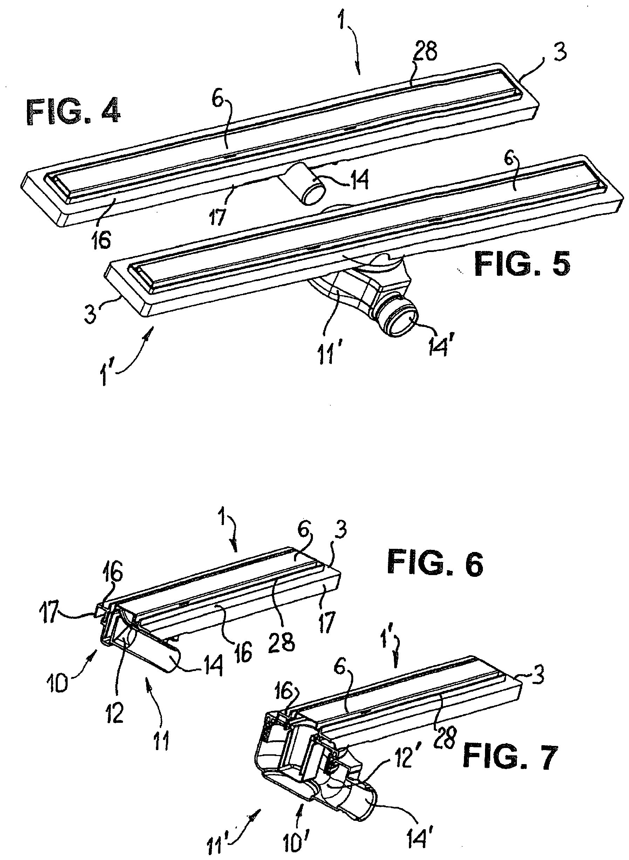

Das in Fig. 1 gezeigte Rinnenmodul 3 ist lösbar verbunden mit einem Geruchsverschlusssystem 11 und stellt eine modulare Wasserablaufvorrichtung 1 dar. An das Geruchsverschlusssystem 11 ist ein Ablaufstutzen 14 angeschlossen. Dieselbe Wasserablaufvorrichtung 1, jedoch mit einer eingesetzten Rinnenabdeckung 6 zeigen Figuren 4 und 6.

Allerdings kann das Ablaufsystem in das Rinnenmodul 3 stofflich integriert sein, d. h. einen nicht lösbaren Verbund bilden, wenn beide Teile beispielsweise in einem Materialstück aus thermoplastischem Kunststoff hergestellt sind. Eine solche Ausführung ist in Fig. 3 gezeigt.

Die modulare Bauweise der Wasserablaufvorrichtung erlaubt es, an das Rinnenmodul 3 ein anderes Ablaufsystem in Form eines bekannten Standardablaufs anzuschließen, wie es in Figuren 5 und 7 dargestellt ist.

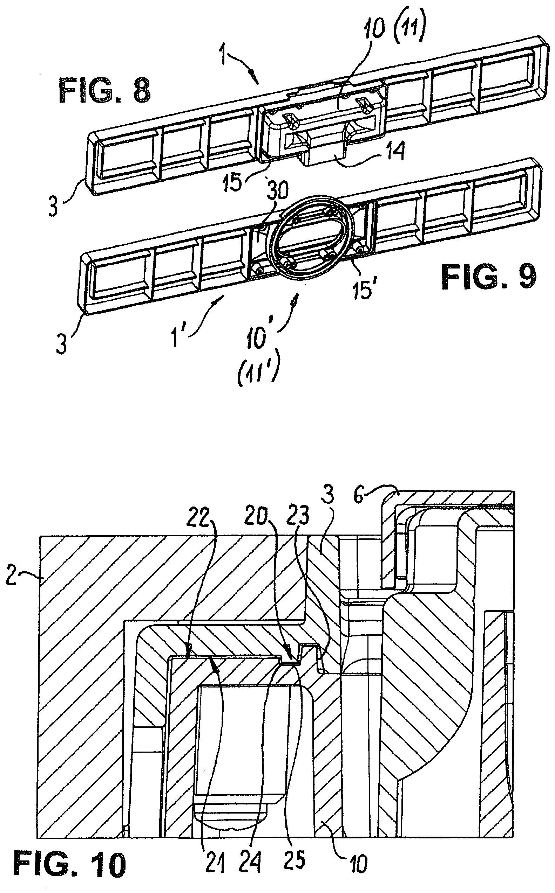

Der modulare Aufbau ist auch in den Figuren 6, 7 und insbesondere in den Figuren 8 und 9 gezeigt. Gemäß der Figuren 6 und 8 weist die Wasserablaufvorrichtung 1 das besagte Rinnenmodul 3 und ein Ablaufmodul 10 mit einem Wassereinlauf 12 auf, wobei das Ablaufmodul 10 das Geruchsverschlusssystem 11 beinhaltet. Dementsprechend ist die in den Figuren 7 und 9 abgebildete Wasserablaufvorrichtung 1' mit einem einen Wassereinlauf 12' aufweisenden Ablaufmodul 10' für einen Standardablauf ausgestattet, wobei das Ablaufmodul 10' das Geruchsverschlusssystem 11' beinhaltet. Das Ablaufmodul 10' läuft in einen Ablaufstutzen 14' aus.

Wie den Figuren 8 und 9 zu entnehmen ist, weisen die Ablaufmodule 10; 10' jeweils eine zur in Fig. 1 dargestellten Wasserauslauföffnung 5 kompatible, rechteckige Kontur 15 oder entsprechend 15' auf. Ferner ist in Fig. 9 ein Adapter 30 zu sehen, mit dem sich eine lösbare Verbindung mit dem Rinnenmodul 3 und dem Geruchsverschlusssystem 11 oder 11' (in Fig. 9 ist nur ein runder Ablauftopf des Standardablaufs gezeigt) realisieren lässt. Der Adapter 30 ist sowohl mit dem Rinnemodul 3 als auch mit dem Wassereinlauf 12; 12' des Ablaufmoduls 10; 10' wasserdicht verbindbar.

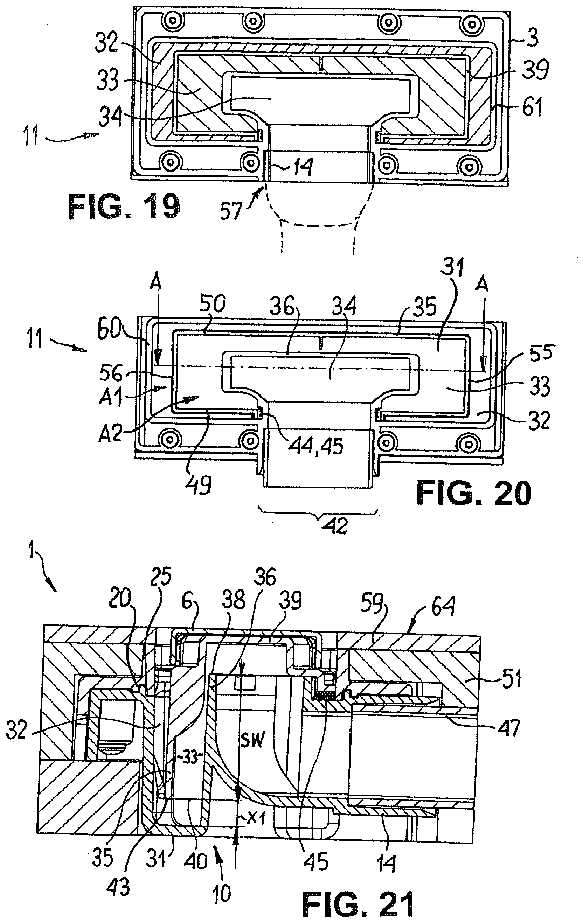

Wie in den Figuren 10 und 21 dargestellt, ist zwischen dem Rinnenmodul 3 und dem Ablaufmodul 10 eine Verbindung 20 vorgesehen, welche durch an Kontaktflächen 21; 22 des Rinnenmoduls und des Ablaufmoduls eingebrachte Strukturelemente 23; 24 entstanden ist. Nach der Herstellung der Verbindung 20 wird ein Dichtungslabyrinth 25 gebildet. Die Fig. 21 zeigt auch die Rinnenabdeckung 6, welche mit einer Trittfläche 64 von auf eine Grundplatte 51 verlegten Fliesen 59 in Flucht liegt.

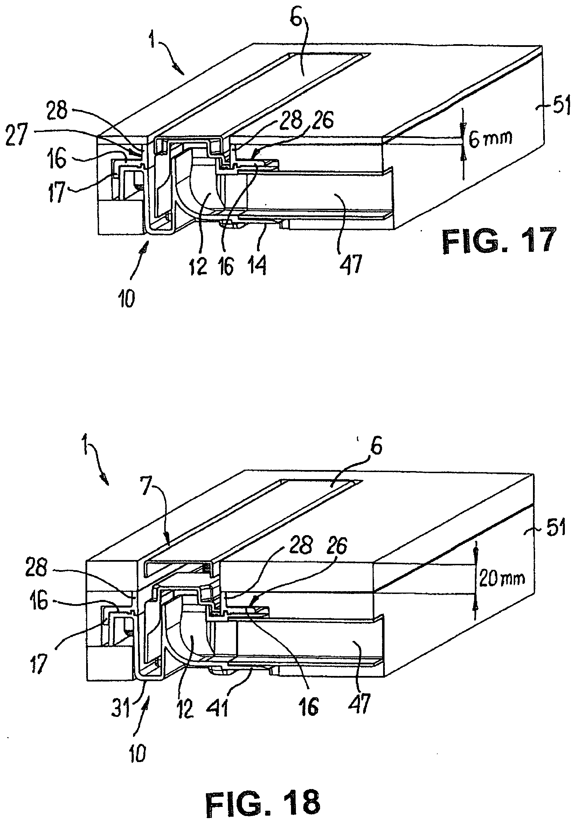

Wie die Figuren 17 und 18 zeigen, kann die Rinnenabdeckung 6 der in die Grundplatte 51 eingebauten Wasserablaufvorrichtung 1 an entsprechende Fliesenstärke angepasst werden. Bei der Fig. 17 handelt es sich um eine 6 mm und bei der Fig. 18 um eine 20 mm Aufbauhöhe. Die Wasserablaufvorrichtung 1 verfügt über eine nicht gezeigte Höhenverstellvorrichtung.

Ferner zeigen die Figuren 6,17 und 18 den bereits in Fig. 1 gezeigten, nach oben ragenden Kragen 28 des Rinnenmoduls 3, an den sich eine waagerechte, streifenförmige Wandung 16 mit einem nach unten zeigenden L-Schenkel 17 anschließt. Die waagerechte Wandung 16 weist eine Auflagefläche 26 und der Kragen 28 entsprechend eine Auflagefläche 26 auf, mit denen sich das Rinnenmodul 3 an der Unterseite U der Grundplatte 51 im Bereich derer Aussparung 52 befestigen, vorzugsweise verkleben lässt. Dabei weist der Kragen 28 Ausmaße auf, die die der Aussparung 52 nicht überschreiten, so dass im montierten Zustand der Kragen 28 in die Aussparung 52 hineinragt und die Grundplatte 51 auf der waagerechten Auflagefläche 26 der Wandung 16 aufliegt. Die Verbindung des Rinnenmoduls 3 mit der Grundplatte 51 ist wasserdicht.

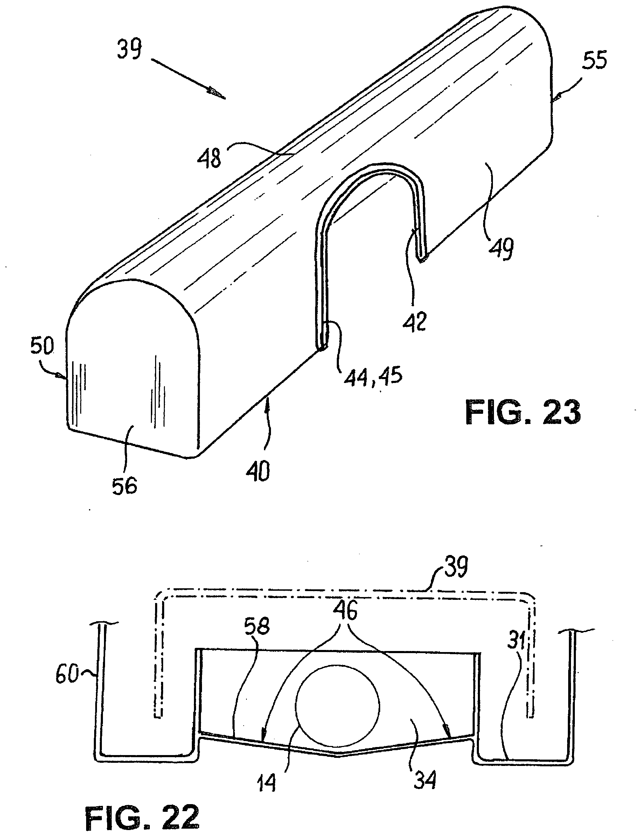

Das Geruchsverschlusssystem 11 ist der Fig. 21 und insbesondere den Figuren 19, 20 und 22 zu entnehmen. Demnächst beinhaltet das Geruchsverschlusssystem 11 ein Gehäuse 60 in Form einer Auffangwanne mit einem Boden 31 (vgl. Figuren 21 und 22), welche in eine innere Kammer 34 und eine äußere Kammer 32 unterteilt ist. Außerdem beinhaltet das Geruchsverschlusssystem 11 ein in das Gehäuse 60 eintauchendes, nach unten offenes (Öffnung 40) Glockenelement 39, welches in seiner einfachsten Ausführungsform detailliert in Fig. 23 gezeigt ist. Die äußere Kammer 32 ist durch eine Gehäusewand 61 (vgl. Fig. 19) begrenzt.

Gemäß Fig. 23 weist das Glockenelement 39 einen langgestreckten, geradlinig verlaufenden Scheitelbereich 48, zwei längliche Seitenwände 49; 50 und zwei kürzere Seitenwände 55, 56 auf, wobei an einer der länglichen Seitenwände (49) eine U-förmige, profilierte Aussparung 42 mit einer Nut 44 eingebracht ist. In die Nut 44 ist ein Dichtelement 45 eingesetzt. Im montierten Zustand ist der Ablaufstutzen 14 dichtend von der Aussparung 42 umschlossen. Das Glockenelement 39 ist in vorliegenden Fall aus Kunststoff hergestellt.

Beim eingesetzten Glockenelement 39 entsteht eine zusätzliche, mittlere Kammer 33. Wie die Figuren 19 und 20 zeigen, ist die innere Kammer 34 in Draufsicht auf den Boden 31 durch eine umlaufende, erste Sperrwand 36 gebildet und gänzlich von der mittleren Kammer 33, ausgenommen Durchgangsbereich 57 zum Ablaufstutzen 14, umschlossen.

Die mittlere Kammer 33 ist ebenso - in Draufsicht auf den Boden 31 - gänzlich von einer durch die Seitenwände 49; 50; 55, 56 gebildeten, zweiten Sperrwand 35, ausgenommen Durchgangsbereich 57 umschlossen. So entsteht eine neuartige Kammer-Anordnung, bei der die Kammern 34, 33, 32 durch ringförmig umeinander herum liegende Sperrwände 36, 35 und die Gehäusewand 61 gebildet sind.

Wie in Fig. 19 mit Strichlinie angedeutet, kann der Durchgangsbereich 57 trichterförmig gestaltet sein.

Gemäß Fig. 22 weist die innere Kammer 34 einen Boden 58 auf, welcher sich von dem übrigen Boden 31 des Gehäuses 60 anhebt. Der Boden 58 der inneren Kammer 34 weist ein dem Ablaufstutzen 14 beidseitig zulaufendes Gefälle 46 auf.

Die äußere Kammer 32 weist auf Höhe einer Unterkante 43 der Sperrwand 35 eine erste Fläche A1 und die mittlere Kammer 33 eine zweite Fläche A2 (vgl. Fig. 20) auf, wobei das Flächenverhältnis von A1 zu A2 in Hinblick auf bauraumoptimierte Gestaltung in vorliegendem Fall 0,66 beträgt.

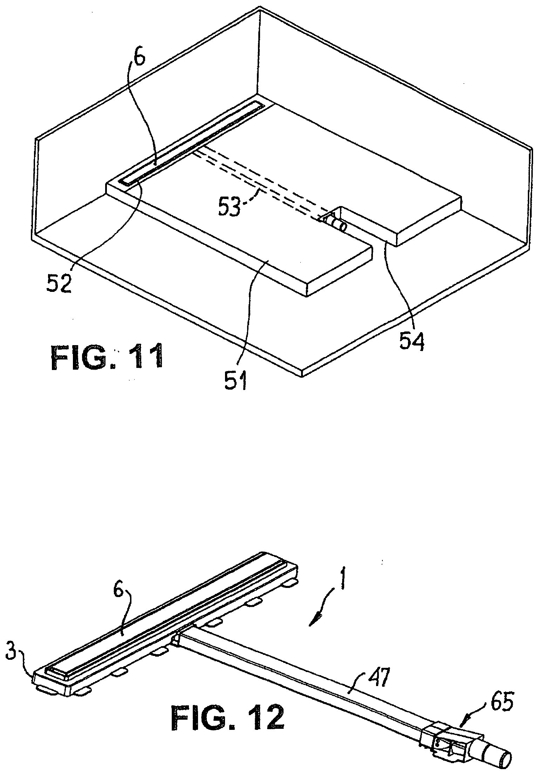

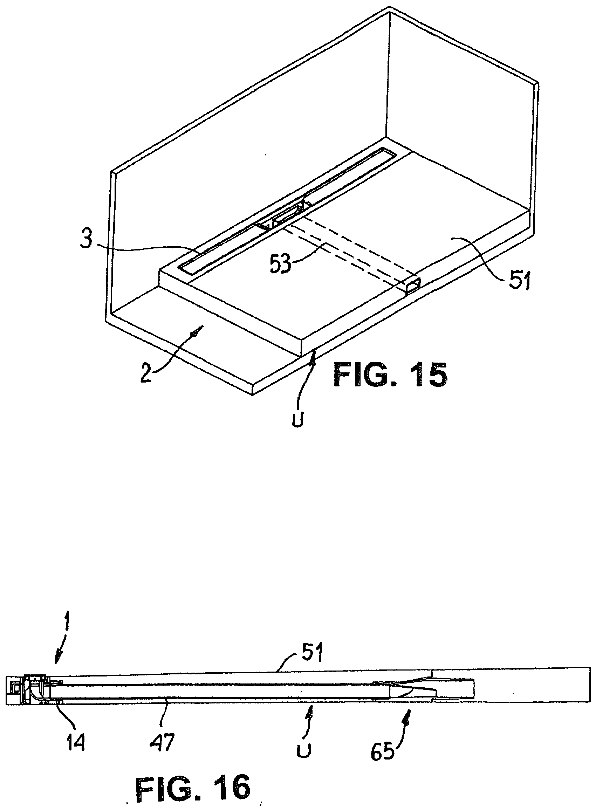

Die in Figuren 12 und 16 schematisch dargestellte Wasserablaufvorrichtung 1 beinhaltet das Rinnenmodul 3 mit Rinnenabdeckung 6, das in Figur nicht gezeigte Geruchsverschlusssystem 11, den Ablaufstutzen 14 und ein vierkantiges Ablaufrohr 47, welches über ein Adapterstück 65 an ein weiteres Ablaufrohr der häuslichen Abwasserinstallation anschließbar ist. Das vierkantige Ablaufrohr 47 ist innerhalb einer in der Grundplatte 51 auf deren Unterseite U gefrästen, nutartigen Aussparung 53 geführt, wobei die letztere in eine die Grundplatte 51 komplett durchdringende Aussparung 54 (vgl. Fig. 11) mündet. Das Ablaufrohr 47 weist ausgehend vom Ablaufstutzen 14 ein Gefälle von etwa 0,5% auf.

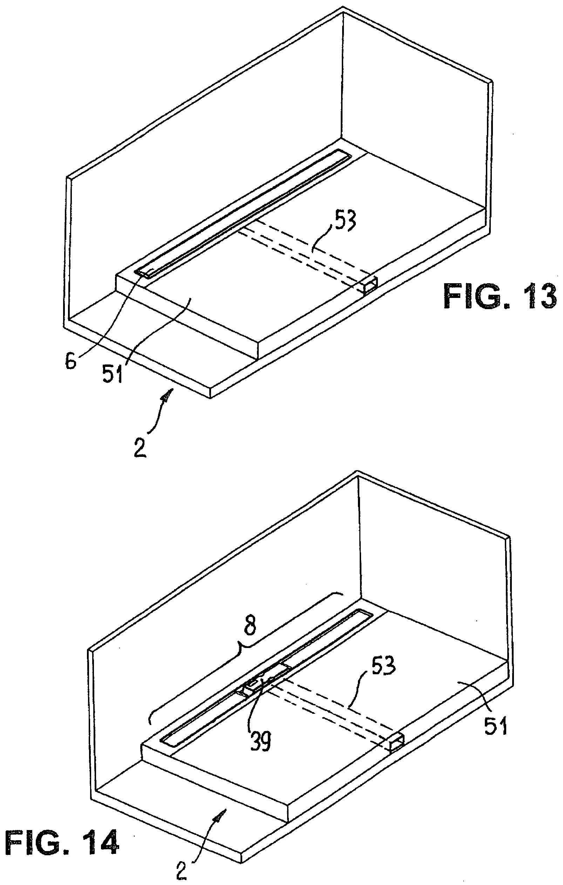

Die Figuren 13, 14 und 15 zeigen ein bodenebenes Duschelement 2, bestehend aus der Grundplatte 51 und der in die Grundplatte 51 eingebauten Wasserablaufvorrichtung 1. An der Grundplatte 51 ist noch eine weitere Aussparung 52 zur Aufnahme der Wasserablaufvorrichtung 1 mit der Rinnenabdeckung (6) vorgesehen, wobei besonders vorteilhaft ist, dass die Wasserablaufvorrichtung 1 möglichst nah an der Wand platziert ist. In Fig. 14 ist der rinnenförmige Ablaufbereich 8 nach Entnahme der Rinnenabdeckung 6 und in Fig. 15 nach dem Herausnehmen des Glockenelementes 39 gezeigt.

### Funktionsweise:

Im montierten Zustand wird ein vorbestimmter Abstand X1 zwischen dem Boden 31 und der Unterkante 43 der Sperrwand 35 (vgl. Fig. 21) gewährleistet. Dabei ist ein Abstand zwischen einer Oberkante 38 der Sperrwand 36 und der Unterkante 43 der Sperrwand 35 zu beachten, welcher in vorliegendem Fall mindestens 38 mm betragen soll. Der Abstand definiert eine Sperrwasserhöhe SW.

Die Flussrichtung des Abwassers ist von der äußeren Kammer 32 zur mittleren Kammer 33, von dort zur inneren Kammer 34 und weiter zum Ablaufstutzen 14 und anschließend zum Ablaufrohr 47 gerichtet.

**Bezugszeichenliste:**

| | |
|---|---|
| 1 | Wasserablaufvorrichtung |
| 1' | Wasserablaufvorrichtung |
| 2 | Duschelement |
| 3 | Rinnenmodul |
| 4 | Wassereinlauföffnung |
| 5 | Wasserauslauföffnung |
| 6 | Rinnenabdeckung |
| 7 | - |
| 8 | rinnenförmiger Ablaufbereich |
| 9 | Übergangsbereich |
| 10 | Ablaufmodul |
| 10' | Ablaufmodul |
| 11 | Geruchsverschlusssystem |
| 11' | Geruchsverschlusssystem |
| 12 | Wassereinlauf |
| 12' | Wassereinlauf |
| 13 | - |
| 14 | Ablaufstutzen |
| 14' | Ablaufstutzen |
| 15 | Kontur |
| 15' | Kontur |
| 16 | Wandung (waagerecht) |
| 17 | L-Schenkel (v. 26) |
| 18 | - |
| 19 | - |
| 20 | Verbindung |
| 21 | Kontaktfläche |
| 22 | Kontaktfläche |
| 23 | Strukturelement |
| 24 | Strukturelement |
| 25 | Dichtungslabyrinth |
| 26 | Auflagefläche (v. 16) |
| 27 | Auflagefläche (v. 28) |
| 28 | Kragen |
| 29 | - |
| 30 | Adapter |
| 31 | Boden |
| 32 | äußere Kammer |
| 33 | mittlere Kammer |
| 34 | innere Kammer |
| 35 | Sperrwand |
| 36 | Sperrwand |
| 37 | - |
| 38 | Oberkante |
| 39 | Glockenelement |
| 40 | Öffnung |
| | |
| 42 | Aussparung |
| 43 | Unterkante |
| 44 | Nut |
| 45 | Dichtelement |
| 46 | Gefälle |
| 47 | Auslaufrohr |
| 48 | Scheitelbereich |
| 49 | Seitenwand |
| 50 | Seitenwand |
| 51 | Grundplatte |
| 52 | Aussparung |
| 53 | Aussparung |
| 54 | Aussparung |
| 55 | Seitenwand |
| 56 | Seitenwand |
| 57 | Durchgangsbereich |
| 58 | Boden |
| 59 | Fliese |
| 60 | Gehäuse |
| 61 | Gehäusewand |
| 62 | Gefälle |
| 63 | - |
| 64 | Trittfläche |
| 65 | Adapterstück |
| | |
| A - A | Schnitt |
| A1 | Fläche |
| A2 | Fläche |
| X1 | Abstand |
| SW | Sperrwasserhöhe |
| U | Unterseite |

## Patentansprüche

1. Wasserablaufvorrichtung (1) für eine sanitäre Anlage, wie einen bodenebenen Duschbereich, umfassend:
- eine Rinnenabdeckung (6),
- einen rinnenförmigen Ablaufbereich (8) mit einer in dessen Mitte angeordneten Wasserauslauföffnung (5) und einer der Wasserauslauföffnung (5) zulaufenden Wassereinlauföffnung (4),
- einen innerhalb des rinnenförmigen Ablaufbereichs (8) angeordneten Übergangsbereich (9) mit einem Geruchsverschlusssystem (11) und wenigstens einem Ablaufstutzen (14) für den Wasseraustritt, wobei das Geruchsverschlusssystem ein Gehäuse (60) mit einer inneren Kammer (34) und wenigstens einer äußeren Kammer (32) sowie ein in das Gehäuse (60) eintauchendes Glockenelement (39) umfasst, wobei die äußere Kammer (32) wenigstens teilweise durch eine Gehäusewand (61) begrenzt ist,
- wobei das Glockenelement (39) ein Hohlkörper ist,
- wobei die innere Kammer (34) in Draufsicht auf einen Boden (31) des Gehäuses (60) durch eine umlaufende, erste Sperrwand (36) gebildet und gänzlich von der äußeren Kammer (32), ausgenommen im Durchgangsbereich (57) zum Ablaufstutzen (14), umschlossen ist,
- wobei zwischen der inneren Kammer (34) und der äußeren Kammer (32) - beim eingesetzten Glockenelement (39) - eine mittlere Kammer (33) angeordnet ist,
- wobei die äußere Kammer (32) durch die zweite Sperrwand (35) von der mittleren Kammer (33) und die mittlere Kammer (33) durch die erste Sperrwand (36) von der inneren Kammer (34) getrennt ist und die Flussrichtung des Abwassers von der äußeren Kammer (32) zur mittleren Kammer (33), von dort zur inneren Kammer (34) und von dort zum Ablaufstutzen (14) gerichtet ist,
**dadurch gekennzeichnet, dass**
- das Glockenelement (39) einen langgestreckten, geradlinig verlaufenden Scheitelbereich (48), zwei ebenso langgestreckte Seitenwände (49, 50) und zwei an den Schmalseiten des Hohlkörpers liegende, weitere Seitenwände (55, 56) aufweist, die eine zweite Sperrwand (35) bilden,
- die mittlere Kammer (33) in Draufsicht auf den Boden (31) gänzlich von der zweiten Sperrwand (35), ausgenommen im Durchgangsbereich (57) zum Ablaufstutzen (14) umschlossen ist, so dass die Kammern (34, 33, 32) durch ringförmig umeinander herum liegende Sperrwände (36, 35) und die Gehäusewand (61) gebildet sind, und
- wobei die Sperrwand (35) des Glockenelementes (39) eine Aussparung (42) aufweist, so dass das Glockenelement (39) über die innere Kammer (34) und den von der inneren Kammer (34) ausgehenden Ablaufstutzen (14) stülpbar ist.

2. Wasserablaufvorrichtung (1) nach Anspruch 1, **dadurch gekennzeichnet, dass**
- das Glockenelement (39) eine zum Boden (31) gerichtete Öffnung (40) aufweist;
- im montierten Zustand die Aussparung (42) den Ablaufstutzen (14) dichtend umschließt und
- im montierten Zustand ein Abstand (X1) zwischen dem Boden (31) und einer Unterkante (43) der Sperrwand (35) vorgesehen ist.

3. Wasserablaufvorrichtung (1) nach Anspruch 1 oder 2, **dadurch gekennzeichnet, dass** die Aussparung (42) durch ein Dichtelement (45) gegenüber dem Ablaufstutzen (14) abgedichtet ist.

4. Wasserablaufvorrichtung (1) nach einem der Ansprüche 1 bis 3, **dadurch gekennzeichnet, dass** auf Höhe der Unterkante (43) der Sperrwand (35) die äußere Kammer (32) eine erste Fläche (A1) und die mittlere Kammer (33) eine zweite Fläche (A2) aufweist, wobei das Flächenverhältnis von (A1) zu (A2) innerhalb eines Bereiches von 0,5 bis 0,8, vorzugsweise zwischen 0,63 und 0,69 liegt.

5. Wasserablaufvorrichtung (1) nach einem der vorhergehenden Ansprüche, **dadurch gekennzeichnet, dass** ein Abstand zwischen der Oberkante (38) der Speerwand (36) und der Unterkante (43) der Sperrwand (35), welcher eine Sperrwasserhöhe (SW) definiert, mindestens 38 mm beträgt.

6. Wasserablaufvorrichtung (1) nach einem der vorhergehenden Ansprüche, **dadurch gekennzeichnet, dass** die innere Kammer (34) einen Boden (58) mit einem dem Ablaufstutzen (14) zugerichteten Gefälle (46) aufweist.

7. Wasserablaufvorrichtung (1) nach Anspruch 6, **dadurch gekennzeichnet, dass** der Boden (58) der inneren Kammer (34) gegenüber dem Boden (31) des Gehäuses (60) angehoben ist.

8. Wasserablaufvorrichtung (1) nach einem der vorhergehenden Ansprüche, **dadurch gekennzeichnet, dass** der Durchgangsbereich (57) der inneren Kammer (34) zum Ablaufstutzen (14) trichterförmig gestaltet ist.

9. Wasserablaufvorrichtung (1) nach einem der vorhergehenden Ansprüche, **dadurch gekennzeichnet, dass** der Ablaufstutzen (14) mit einem Ablaufrohr (47) verbunden ist.

10. Wasserablaufvorrichtung (1) nach einem der vorhergehenden Ansprüche, **dadurch gekennzeichnet, dass** das Ablaufrohr (47) einen rechteckeigen, runden oder ovalen Querschnitt aufweist.

11. Wasserablaufvorrichtung (1) nach einem der Ansprüche 1 bis 10, **dadurch gekennzeichnet, dass** die Wasserablaufvorrichtung (1) ein Rinnenmodul (3) mit einer Wasserauslauföffnung (5) und ein Ablaufmodul (10) mit einem Wassereinlauf (12; 12') umfasst, wobei
- das Ablaufmodul das Geruchsverschlusssystem (11) beinhaltet,
- die Wasserauslauföffnung (5) des Rinnenmoduls (3) rechteckig oder oval ist,
- der Wassereinlauf (12; 12') des Geruchsverschlusssystems (11) eine zur Wasserauslauföffnung (5) kompatible Kontur (15; 15') aufweist,
- zwischen dem Rinnenmoduls (3) und dem Ablaufmodul (10) eine Verbindung (20) vorgesehen ist.

12. Wasserablaufvorrichtung (1) nach Anspruch 11, **dadurch gekennzeichnet, dass** es sich bei der Verbindung (20) des Rinnenmoduls (3) mit dem Ablaufmodul (10) um eine Kleb-, Klick-, Rast- oder Schraubverbindung handelt.

13. Wasserablaufvorrichtung (1) nach Anspruch 11 oder 12, **dadurch gekennzeichnet, dass** das Rinnenmoduls (3) und das Ablaufmodul (10) Kontaktflächen (21; 22) und Strukturelemente (23; 24) aufweisen, wobei die Strukturelemente (23; 24) so angelegt sind, dass sie nach Herstellung der Verbindung (20) ein Dichtungslabyrinth (25) ausbilden.

14. Wasserablaufvorrichtung (1) nach einem der Ansprüche 11 bis 13, **dadurch gekennzeichnet, dass** das Ablaufmodul (10; 10') wenigstens einen Adapter (30) zur lösbaren Verbindung mit dem Rinnenmodul (3) und dem Geruchsverschlusssystem (11; 11') umfasst, wobei der Adapter (30) mit dem Rinnemodul (3) und dem Wassereinlauf (12; 12') des Ablaufmoduls (10; 10') wasserdicht verbindbar ist.

15. Bodenebenes Duschelement (2) mit einer Wasserablaufvorrichtung (1; 1') nach einem der vorhergehenden Ansprüche, bei dem die Wasserablaufvorrichtung (1; 1') einen rinnenförmiges Ablaufbereich (8) und einen zentralen Übergangsbereich (9) mit einem Geruchsverschlusssystem (11; 11') und wenigstens einem Ablaufstutzen (14, 14') umfasst;
**dadurch gekennzeichnet, dass**:
- an den Ablaufstutzen (14; 14') des Geruchsverschlusssystem (11; 11') sich ein Ablaufrohr (47) anschließt,
- das Ablaufrohr (47) ausgehend vom Ablaufstutzen (14; 14') ein Gefälle zwischen 0,5% und 1 % aufweist,
- zur Aufnahme des Ablaufrohres (47) eine Grundplatte (51) vorgesehen ist, welche an ihrer Unterseite (U) eine nutartige Aussparung (53) aufweist,
- die nutartige Aussparung (53) in eine die Grundplatte (51) komplett durchdringende Aussparung (54) mündet,
- die Grundplatte (51) eine weitere Aussparung (52) zur Aufnahme der Wasserablaufvorrichtung (1; 1') mit dem rinnenförmigen Ablaufbereich (8) aufweist
- und die Wasserablaufvorrichtung (1; 1'), ausgenommen Rinnenabdeckung (6) in die Unterseite (U) der Grundplatte (51) eingelassen und dort festgelegt ist.

## Claims

1. Water-drainage device (1) for a sanitary installation, such as a floor-level shower area, comprising:
- a channel covering (6),
- a channel-like drainage region (8) having a centrally arranged water-outflow opening (5) and a water-inflow opening (4), which runs towards the water-outflow opening (5),
- a transition region (9), which is arranged within the channel-like drainage region (8) and has an odour-seal system (11) and at least one drainage connector (14) through which water can exit, wherein the odour-seal system comprises a housing (60), with an inner chamber (34) and at least one outer chamber (32), and a bell element (39), which penetrates into the housing (60), wherein the outer chamber (32) is delimited, at least in part, by a housing wall (61),
- wherein the bell element (39) is a hollow body,
- wherein, as seen in plan view of a floor (31) of the housing (60), the inner chamber (34) is formed by an encompassing, first blocking wall (36) and, with the exception of the through-passage region (57) to the drainage connector (14), is enclosed entirely by the outer chamber (32),
- wherein a central chamber (33) is arranged between the inner chamber (34) and the outer chamber (32) - with the bell element (39) inserted,
- wherein the outer chamber (32) is separated by the second blocking wall (35) from the central chamber (33) and the central chamber (33) is separated by the first blocking wall (36) from the inner chamber (34), and the flow direction of the waste water is directed from the outer chamber (32) to the central chamber (33), from there to the inner chamber (34), and from there to the drainage connector (14),
**characterized in that**
- the bell element (39) has an elongate, rectilinearly running apex region (48), two likewise elongate side walls (49, 50) and two further side walls (55, 56), which are located on the narrow sides of the hollow body and form a second blocking wall (35),
- as seen in plan view of the floor (31), the central chamber (33), with the exception of the through-passage region (57) to the drainage connector (14), is enclosed entirely by the second blocking wall (35), and therefore the chambers (34, 33, 32) are formed by the housing wall (61) and by blocking walls (36, 35) situated around one another in ring form, and
- wherein the blocking wall (35) of the bell element (39) has an aperture (42), and therefore the bell element (39) can be fitted over the inner chamber (34) and the drainage connector (14), which extends from the inner chamber (34).

2. Water-drainage device (1) according to Claim 1, **characterized in that**
- the bell element (39) has an opening (40) directed towards the floor (31);
- in the installed state, the aperture (42) encloses the drainage connector (14) with sealing action; and
- in the installed state, a distance (X1) is provided between the floor (31) and a lower edge (43) of the blocking wall (35).

3. Water-drainage device (1) according to Claim 1 or 2, **characterized in that** the aperture (42) is sealed in relation to the drainage connector (14) by a sealing element (45).

4. Water-drainage device (1) according to one of Claims 1 to 3, **characterized in that**, level with the lower edge (43) of the blocking wall (35), the outer chamber (32) has a first surface (A1) and the central chamber (33) has a second surface (A2), wherein the area ratio of (A1) to (A2) ranges from 0.5 to 0.8, and is preferably between 0.63 and 0.69.

5. Water-drainage device (1) according to one of the preceding claims, **characterized in that** the upper edge (38) of the blocking wall (36) and the lower edge (43) of the blocking wall (35) are spaced apart by a distance which defines a blocking-water height (SW) and is at least 38 mm.

6. Water-drainage device (1) according to one of the preceding claims, **characterized in that** the inner chamber (34) has a floor (58) with a downward slope (46) which is directed towards the drainage connector (14).

7. Water-drainage device (1) according to Claim 6, **characterized in that** the floor (58) of the inner chamber (34) is raised in relation to the floor (31) of the housing (60).

8. Water-drainage device (1) according to one of the preceding claims, **characterized in that** the through-passage region (57) of the inner chamber (34) to the drainage connector (14) is of funnel-like configuration.

9. Water-drainage device (1) according to one of the preceding claims, **characterized in that** the drainage connector (14) is connected to a drainage pipe (47).

10. Water-drainage device (1) according to one of the preceding claims, **characterized in that** the drainage pipe (47) is of a rectangular, round or oval cross section.

11. Water-drainage device (1) according to one of Claims 1 to 10, **characterized in that** the water-drainage device (1) comprises a channel module (3) with a water-outflow opening (5) and also a drainage module (10) with a water inflow (12; 12'), wherein
- the drainage module contains the odour-seal system (11),
- the water-outflow opening (5) of the channel module (3) is rectangular or oval,
- the water inflow (12; 12') of the odour-seal system (11) has a contour (15; 15') which is compatible with the water-outflow opening (5),
- a connection (20) is provided between the channel module (3) and the drainage module (10).

12. Water-drainage device (1) according to Claim 11, **characterized in that** the connection (20) between the channel module (3) and the drainage module (10) is an adhesive-bonding connection, a click-fit connection, a latching connection or a screw connection.

13. Water-drainage device (1) according to Claim 11 or 12, **characterized in that** the channel module (3) and the drainage module (10) have contact surfaces (21; 22) and structural elements (23; 24), wherein the structural elements (23; 24) are applied such that, once the connection (20) has been established, they form a labyrinth seal (25).

14. Water-drainage device (1) according to one of Claims 11 to 13, **characterized in that** the drainage module (10; 10') comprises at least one adapter (30) for releasable connection to the channel module (3) and the odour-seal system (11; 11'), wherein the adapter (30) can be connected in a water-tight manner to the channel module (3) and the water inflow (12; 12') of the drainage module (10; 10').

15. Floor-level shower element (2) having a water-drainage device (1; 1') according to one of the preceding claims, in the case of which the water-drainage device (1; 1') comprises a channel-like drainage region (8) and a central transition region (9) having an odour-seal system (11; 11') and at least one drainage connector (14, 14';)
**characterized in that**:
- the drainage connector (14; 14') of the odour-seal system (11; 11') is adjoined by a drainage pipe (47),
- starting from the drainage connector (14; 14'), the drainage pipe (47) has a downward slope between 0.5% and 1%,
- a baseplate (51), which has a groove-like aperture (53) on its underside (U), is provided for the purpose of accommodating the drainage pipe (47),
- the groove-like aperture (53) opens out into an aperture (54) which passes all the way through the baseplate (51),
- the baseplate (51) has a further aperture (52) for accommodating the water-drainage device (1; 1') with the channel-like drainage region (8),
- and with the exception of the channel covering (6), the water-drainage device (1; 1') is let into the underside (U) of the baseplate (51) and secured there.

## Revendications

1. Ensemble (1) d'évacuation d'eau pour installation sanitaire, par exemple fond plan de douche, l'ensemble comprenant :
un recouvrement (6) de rigole,
une partie (8) d'évacuation en forme de rigole présentant une ouverture (5) de sortie d'eau disposée en son milieu et une ouverture (4) d'entrée d'eau qui alimente l'ouverture (5) de sortie d'eau,
une partie de transition (9) disposée à l'intérieur de la partie (8) d'évacuation en rigole et présentant un système coupe-odeur (11) et au moins une tubulure d'évacuation (14) pour la sortie d'eau,
le système coupe-odeur comprenant un boîtier (60) doté d'une chambre intérieure (34) et d'au moins une chambre extérieure (32) ainsi qu'un élément (39) en cloche qui s'enfonce dans le boîtier (60),
la chambre extérieure (32) étant délimitée au moins en partie par une paroi (61) du boîtier,
l'élément (39) en cloche étant un corps creux,
la chambre intérieure (34) étant formée dans une vue en plan du fond (31) du boîtier (60) par une première paroi périphérique de séparation (36) et étant entièrement entourée par la chambre extérieure (32) reprise dans la partie de passage (57) conduisant à la tubulure d'évacuation (14),
une chambre intermédiaire (33) étant disposée entre la chambre intérieure (34) et la chambre extérieure (32) lorsque l'élément (39) en cloche a été placé,
la chambre extérieure (32) étant séparée par la deuxième paroi de séparation (35) de la chambre intermédiaire (33) et la chambre intermédiaire (33) étant séparée de la chambre intérieure (34) par la première paroi de séparation (36),
la direction d'écoulement de l'eau usée allant de la chambre extérieure (32) à la chambre intermédiaire (33) et de là vers la chambre intérieure (34) et ensuite la tubulure d'évacuation (14),
**caractérisé en ce que**
l'élément (39) en cloche présente une partie (48) en sommet allongé s'étendant en ligne droite, deux parois latérales (49, 50) également allongées et deux autres parois latérales (55, 56) situées sur les côtés étroits du corps creux et qui forment une deuxième paroi de séparation (35),
**en ce que** dans une vue en plan du fond (31), la chambre intermédiaire (33) est entourée complètement par la deuxième paroi de séparation (35), sauf dans la partie de passage (57) conduisant à la tubulure d'évacuation (14) de telle sorte que les chambres (34, 33, 32) sont formées par des parois de séparation (36, 35) qui s'étendent les unes autour des autres en forme d'anneau et la paroi (61) du boîtier et
**en ce que** la paroi de séparation (35) de l'élément (39) en cloche présente une découpe (42) de telle sorte que l'élément (39) en cloche peut être enfiché au-dessus de la chambre intérieure (34) et de la tubulure d'évacuation (14) qui part de la chambre intérieure (34).

2. Ensemble (1) d'évacuation d'eau selon la revendication 1, **caractérisé en ce que** l'élément (39) en cloche présente une ouverture (40) orientée vers le fond (31),
**en ce qu'**à l'état monté, la découpe (42) entoure de manière étanche la tubulure d'évacuation (14) et
**en ce qu'**à l'état monté, une distance (X1) est prévue entre le fond (31) et le bord inférieur (43) de la paroi de séparation (35).

3. Ensemble (1) d'évacuation d'eau selon les revendications 1 ou 2, **caractérisé en ce que** la découpe (42) est séparée de manière hermétique de la tubulure d'évacuation (14) par un élément d'étanchéité (45).

4. Ensemble (1) d'évacuation d'eau selon l'une des revendications 1 à 3, **caractérisé en ce qu'**à hauteur du bord inférieur (43) de la paroi de séparation (35), la chambre extérieure (32) d'une première superficie (A1) et la chambre intermédiaire (33) une deuxième superficie (A2), le rapport de superficie entre (A1) et (A2) étant situé à l'intérieur d'une plage de 0,5 à 0,8 et en particulier de 0,63 à 0,69.

5. Ensemble (1) d'évacuation d'eau selon l'une des revendications précédentes, **caractérisé en ce que** la distance entre le bord supérieur (38) de la paroi de séparation (36) et le bord inférieur (43) de la paroi de séparation (35), qui défini la hauteur (SW) du siphon de blocage est d'au moins 38 mm.

6. Ensemble (1) d'évacuation d'eau selon l'une des revendications précédentes, **caractérisé en ce que** la chambre intérieure (34) présente un fond (58) doté d'une pente (46) orientée vers la tubulure d'évacuation (14).

7. Ensemble (1) d'évacuation d'eau selon la revendication 6, **caractérisé en ce que** le fond (58) de la chambre intérieure (34) est relevé par rapport au fond (31) du boîtier (60).

8. Ensemble (1) d'évacuation d'eau selon l'une des revendications précédentes, **caractérisé en ce que** la partie de passage (57) allant de la chambre intérieure (34) à la tubulure d'évacuation (14) a la forme d'un entonnoir.

9. Ensemble (1) d'évacuation d'eau selon l'une des revendications précédentes, **caractérisé en ce que** la tubulure d'évacuation (14) est reliée à un tube d'évacuation (47).

10. Ensemble (1) d'évacuation d'eau selon l'une des revendications précédentes, **caractérisé en ce que** le tube d'évacuation (47) présente une section transversale rectangulaire, circulaire ou ovale.

11. Ensemble (1) d'évacuation d'eau selon l'une des revendications 1 à 10, **caractérisé en ce que** l'ensemble (1) d'évacuation d'eau comporte un module de rigole (3) doté d'une ouverture (5) d'évacuation d'eau et un module d'évacuation (10) doté d'une entrée d'eau (12; 12'),
**en ce que** le module d'évacuation contient le système coupe-odeur (11),
**en ce que** l'ouverture (5) d'évacuation d'eau du module de rigole (3) est de forme rectangulaire ou ovale,
**en ce que** l'entrée (12; 12') d'eau du système coupe-odeur (11) présente un contour (15; 15') compatible avec l'ouverture (5) d'évacuation d'eau et
**en ce qu'**une liaison (20) est prévue entre le module de rigole (3) et le module d'évacuation (10).

12. Ensemble (1) d'évacuation d'eau selon la revendication 11, **caractérisé en ce que** la liaison (20) du module de rigole (3) au module d'évacuation (10) est une liaison collée, une liaison à cliquer, une liaison encliquetée ou une liaison filetée.

13. Ensemble (1) d'évacuation d'eau selon les revendications 11 ou 12, **caractérisé en ce que** le module de rigole (3) et le module d'évacuation (10) présentent des surfaces de contact (21; 22) et des éléments structurels (23; 24), les éléments structurels (23; 24) étant disposés de manière à former un labyrinthe d'étanchéité (25) après réalisation de la liaison (20).

14. Ensemble (1) d'évacuation d'eau selon l'une des revendications 11 à 13, **caractérisé en ce que** le module d'évacuation (10; 10') comporte au moins un adaptateur (30) qui permet une liaison libérable avec le module de rigole (3) et le système coupe-odeur (11; 11'), l'adaptateur (30) pouvant être relié de manière étanche à l'eau au module de rigole (3) et à l'entrée d'eau (12; 12') du module d'évacuation (10; 10').

15. Elément de douche (2) à fond plan présentant un ensemble (1; 1') d'évacuation d'eau selon l'une des revendications précédentes, dans lequel l'ensemble (1; 1') d'évacuation d'eau comporte une partie d'évacuation (8) en forme de rigole et une partie centrale de transition (9) dotée d'un système coupe-odeur (11; 11') et d'au moins une tubulure d'évacuation (14; 14'), **caractérisé en ce que**
un tube d'évacuation (47) se raccorde à la tubulure d'évacuation (14; 14') du système coupe-odeur (11; 11'),
**en ce que** le tube d'évacuation (47) présente une pente comprise entre 0,5 % et 1 % à partir de la tubulure d'évacuation (14; 14'),
**en ce que** pour reprendre le tube d'évacuation (47) est prévue une plaque de base (51) qui présente une découpe (53) en forme de rainure sur son côté inférieur (U),
**en ce que** la découpe (53) en forme de rainure débouche dans une découpe (54) qui traverse complètement la plaque de base (51),
**en ce que** la plaque de base (51) présente une autre découpe (52) qui reprend l'ensemble (1; 1') d'évacuation d'eau avec la partie d'évacuation (8) en forme de rigole et
**en ce que** l'ensemble (1; 1') d'évacuation d'eau est ménagée et fixée dans le côté inférieur (U) de la plaque de base (51), sauf pour le recouvrement (6) de la rigole.
